# EUROPEAN PATENT APPLICATION

(11) **EP 4 108 239 A1**
(43) Date of publication of application: **28.12.2022**
(21) Application number: 22183035.9
(22) Date of filing: 23.05.2016
(51) Int. Cl.: A61K 31/198, A61K 31/135, A61P 25/24, A61K 45/06, A61P 25/18, A61P 25/02

(54) **THERAPEUTIC USES OF L-4-CHLOROKYNURENINE**

(30) Priority: 22.05.2015 US 201562179924 P
(62) Divisional of application: 16800590.8
(71) Applicant: Vistagen Therapeutics, Inc., South San Francisco, CA 94080 (US)
(72) Inventor: Snodgrass, Ralph H., San Jose, CA 95120 (US); Cato, Allen E., Durham, NC 27713 (US); Hicklin, Jack S., San Diego, CA 92121 (US)
(74) Representative: Jacobi, Markus Alexander

(57) **Abstract**

Pharmaceutical compositions comprising L-4-chlorokynurenine and associated therapeutic methods are provided for the treatment of certain neurological and other conditions.

## Description

### CROSS-REFERENCE TO RELATED APPLICATIONS

This application is related to U.S. Provisional Patent Application number 62/179,924, filed May 22, 2015 and titled "Therapeutic Uses of L-4-chlorokynurenine," the content of which is hereby incorporated by reference in its entirety.

### STATEMENT REGARDING FEDERALLY SPONSORED RESEARCH

The data presented in this application was supported at least in part by Grant Number 2R44DA0 185 15-02 from the U.S. National Institutes of Health. The government has certain rights in the invention.

### FIELD OF THE INVENTION

The present invention relates to compositions of L-4-chlorokynurenine (L-4-Cl-KYN) in unit dose form and to the use of these compositions for the treatment of neurological and other conditions.

### BACKGROUND OF THE INVENTION

Glutamic acid is a major excitatory neurotransmitter in the mammalian central nervous system, and it is involved in the regulation of several different pathways. Associations have been reported between excessive endogenous glutamic acid and various neurological disorders, both acute and chronic, such as cerebral ischemia, epilepsy, amyotrophic lateral sclerosis, Huntington's disease, Parkinson's disease and Alzheimer's disease.

Overactive glutamatergic transmission via N-methyl-D-aspartate (NMDA) receptors (NMDA-R) is known to play a key role in several neurologic conditions, such as neuropathic pain for example. However, direct acting (e.g., by channel blocking) NMDA-R antagonists produce a number of side effects, such as psychosis, which have limited their therapeutic utility. Antagonism of NMDA-Rs can also be achieved through blockade of a modulatory site on the NMDA-R, known as the glycine B (GlyB) coagonist site. [Parsons et al. 1997] When compared with classic NMDA-R antagonists, GlyB antagonists have a much better safety profile and do not cause the adverse side effects that are associated with "classic" NMDA-R antagonists. [Carter et al. 1992, Leeson et al. 1994, Rundfeldt et al. 1994]

GlyB antagonists also have been shown to reduce hyperalgesia and allodynia in *ex vivo* and animal neuropathic pain models, and have fewer side effects than classic NMDA-R antagonists, making them a safer alternative as potential analgesics. See, for example, Catarzi et al. 2006.

One of the most potent and specific GlyB antagonists currently known is 7-chlorokynurenic acid (7-Cl-KYNA), which is a synthetic, chlorinated analogue of an endogenous neuromodulator, kynurenic acid. 7-chlorokynurenic acid has been shown to prevent excitotoxic and ischemic neuronal damage but does not cross the blood-brain barrier. Thus, its clinical use is limited. [Kemp et al. 1988, Rao et al. 1993]

In contrast, L-4-chlorokynurenine, a prodrug of 7-chlorokynurenic acid, readily gains access to the central nervous system (CNS) after oral or parenteral administration. [Hokari et al. 1996, Lee et al. 2001, Wu et al. 2002, Wu et al. 2000] L-4-chlorokynurenine is efficiently converted to 7-chlorokynurenic acid within activated astrocytes [Lee et al. 2001] and brain levels of 7-chlorokynurenic acid are increased at sites of neuronal injury or excitotoxic insult as a result of astrocyte activation. [Lee et al. 2001]

In preclinical studies, L-4-chlorokynurenine has shown anti-seizure activity in rats. [Wu et al. 2002]. The compound also was found to increase the firing rate and burst firing activity of dopaminergic neurons in the brains of rats. [Linderholm et al. 2007]

Methods for the synthesis of a class of 4,6-disubstituted kynurenine derivatives and monosubstituted derivatives, including L-4-chlorokynurenine, and their use as antagonists to the NMDA receptor were described in U.S. Patent No. 5,547,991. Pharmaceutical compositions containing these compounds, and their therapeutic use also were described.

### SUMMARY OF THE INVENTION

The invention and its various embodiments are set out in the claims that form part of this patent application.

Without limiting the foregoing, in a preferred aspect, the invention relates to pharmaceutical compositions that per unit dose consist essentially of L-4-chlorokynurenine in an amount of about 360, 1,080 or 1,440 mg, together with pharmaceutically acceptable ingredients such as carriers and excipients. Another aspect of the invention involves the administration of therapeutically effective amounts of these compounds to treat conditions, disorders and diseases described in this specification, preferably certain conditions, disorders and diseases caused by neurological dysfunction.

Another aspect of the invention relates to dosing protocols for the inventive compositions, such as the administration of a daily dose of from about 1 to about 14 or from about 1 to about 30 days, more preferably from about 7 to about 24 days, and most preferably from about 12 to about 16 days.

Another preferred aspect of the invention relates to compositions and methods for the treatment of depression and for the treatment of various types of pain, including hyperalgesia, by administering a therapeutically effective amount of L-4-chlorokynurenine. Other aspects of the invention include the treatment of anxiety, thoughts of hopelessness, self-harm or suicide and/or an absence of positive thoughts or plans, major depressive disorder (MDD), dysthymic disorder (or dysthymia), persistent depressive disorders, atypical depression, bipolar depression or manic depression, seasonal affective disorder (SAD), psychotic depression and postpartum depression, psychotic depression, premenstrual syndrome, premenstrual dysphoric disorder, anxiety, mood disorder, depressions caused by chronic medical conditions such as cancer or chronic pain, chemotherapy, chronic stress, post-traumatic stress disorders, risk of suicide. Such therapies optionally include the co-administration of L-4-chlorokynurenine along with another antidepressant or mood elevating therapy including cognitive and psychotherapy.

In another aspect, the invention relates to pharmaceutical compositions and associated therapeutic methods of administering L-4-chlorokynurenine in an amount that produces plasma levels of 7-chlorokynurenic acid as described in this specification. A preferred aspect of the invention relates to the administration of L-4-chlorokynurenine at a dose sufficient to produce plasma levels of 7-chlorokynurenic acid are in the range of about 15 ng/mL to 550 ng/mL.

Another aspect of the invention relates to a combination pharmaceutical product and associated methods that comprise formulations including L-DOPA and the pharmaceutical composition of L-4-chlorokynurenine described in this application, and to their co-administration, either simultaneously or sequentially at dosages described in the application in order to reduce L-DOPA associated dyskinesias.

Further aspects of the present invention relate to the treatment of tinnitus and of obsessive-compulsive disorder by administering an effective amount of L-4-chlorokynurenine in a pharmaceutical composition. Preferred administration is a daily dose of from one to about 30 days and in a range of about 50 mg to about 1,800 mg per day, more preferably in a daily amount of about 360, 1,080 or 1,440 mg, together with pharmaceutically acceptable ingredients such as carriers and excipients. Other administration protocols are described in this specification. Pharmaceutical compositions comprising L-4-chlorokynurenine packaged with a label indicating the use of this composition for the treatment of a condition selected from the group comprising tinnitus and obsessive-compulsive disorder are contemplated.

### BRIEF DESCRIPTION OF THE DRAWINGS

The following figures, which are described below and which are incorporated in and constitute a part of the specification, illustrate exemplary embodiments according to the disclosure and are not to be considered limiting of the scope of the invention, for the invention may admit to other equally effective embodiments. The figures are not necessarily to scale, and certain features and certain views of the figures may be shown exaggerated in scale or in schematic in the interest of clarity and conciseness.
Figure 1 represents the mean (n = 12 or 13) plasma concentrations of L-4-chlorokynurenine on days 1 and 14 after oral administration of once-daily doses of L-4-chlorokynurenine.
Figure 2 represents the mean (n = 12 or 13) plasma concentrations of 7-chlorokynurenic acid on days 1 and 14 after oral administration of once-daily doses of L-4- chlorokynurenine.

### DETAILED DESCRIPTION OF THE INVENTION

The invention is described below, with reference to detailed illustrative embodiments. It will be apparent that the invention may be embodied in a wide variety of forms, some of which may be quite different from those of the disclosed embodiments. Consequently, the specific structural and functional details disclosed below are merely representative and do not limit the scope of the invention.

In work supported by a grant from the National Institutes of Health, it was shown that L-4- chlorokynurenine (also known as VistaGen AV-101) had potent antihyperalgesic actions in three animal models of peripheral tissue inflammation and nerve injury with no evidence of side effects. Overall, in animal models, L-4-chlorokynurenine was well tolerated, and produced no safety or toxicity at doses that produce antihyperalgesic effects in animal models.

The present invention is based on the inventors' clinical findings that certain doses of L-4-chlorokynurenine are in fact safe and tolerable in humans without substantial side effects or any significant adverse effects. Moreover, it was surprisingly found that at such dosages, subjects reported relief from neuropathic pain, specifically, hyperalgesia pain. It was also surprisingly found that a substantial number of subjects in the clinical study reported positive feelings of "well-being" from the administration of L-4-chlorokynurenine, an indicator of anti-depressive activity, whereas this was not reported by the placebo controls.

The present invention relates to compositions and therapeutic methods to treat various types of neurological disorders, neuropathies (both central and peripheral) and dysfunction such as those caused by: (a) injury and drug toxicities such as result from chemotherapy and anti-viral drugs; (b) diseases and neurodegenerative disorders such as diabetes, cancer, viral infection, Multiple Sclerosis, spondylitis, polyneuritis, surgery, amputation, epilepsy, convulsions, Parkinson's disease, Huntington's disease, and Alzheimer's disease and those diseases and conditions involving overactive glutamatergic transmission via N-methyl-D-aspartate receptors; and (c) imbalances in neurotransmitters, receptors and signaling pathways associated with depression and other psychiatric disorders. The treatment of pain is expressly contemplated, including but not limited to neurogenic pain, spontaneous pain, allodynia hyperalgesia pain, mechanical hyperalgesia pain and heat hyperalgesia pain. Moreover, the treatment of depression is also explicitly contemplated as an aspect of the present invention.

### Definitions:

"Consisting essentially of" a specified amount of a pharmaceutically active agent means that there is no additional amount of that agent. The presence of other ingredients, for example, excipients and/or lubricants, and the like, or different pharmaceutically active ingredients in combination is not precluded. For example, the combination of L-DOPA with 4-chlorokynurenine is expressly contemplated, as are other compositions and methods for their administration that include 4- chlorokynurenine plus another active ingredient that are given either simultaneously or sequentially but in a time frame in which they have substantially the same therapeutic effect as if they had been administered in a single combination pharmaceutical product.

"Pharmaceutical unit dose," "unit dose" or "unit dose form" means a single dose of L-4-chlorokynurenine, which is capable of being administered to a subject, and which can be readily handled and packaged, remaining as a physically and chemically stable unit dose.□

"Therapeutically effective" means that the amount of L-4-chlorokynurenine administered and converted to 7-chlorokynurenic acid acts to down-regulate NMDA-R mediated signal transmission or an imbalance in neurotransmitters that is sufficient to produce a clinical improvement in neurological function, such as a decrease in neuropathic pain, or an increase in feelings of well-being or reduction in depressive mood or feelings.□

"Without significant adverse effect" means that substantially all patients to whom L-4-chlorokynurenine is administered will have no more than a "mild" adverse event as defined by the U.S. Food and Drug Administration (FDA). The FDA defines a "mild adverse event" as an event that is easily tolerated by the subject, causing minimal discomfort and not interfering with everyday activities. In contrast, a "moderate adverse event" is an event that is sufficiently discomforting causing it to interfere with normal everyday activities.

### Pharmaceutical Compositions:

L-4-chlorokynurenine has been synthesized by the methods of U.S. Patent No. 5,547,991. More recent synthesis processes also have been reported in the medical literature, such as Salituro et al. 1994. And preferred synthesis methods are described in published international patent applications WO/2014/152752 and WO/2014/152835. L-4-chlorokynurenine also is available commercially from various sources, including BOC Sciences (Shirley, NY, USA) and Advanced Technology & Industrial Co., Ltd. (Hong Kong, China). Cambridge Major Laboratories (Germantown, WI, USA) manufactured the L-4-chlorokynurenine used in the clinical study discussed in this patent application.

A preferred embodiment of the invention relates to pharmaceutical compositions comprising a unit dose of a therapeutically effective amount of L-4-chlorokynurenine that is formulated for oral administration, together with pharmaceutically acceptable carriers and excipients.

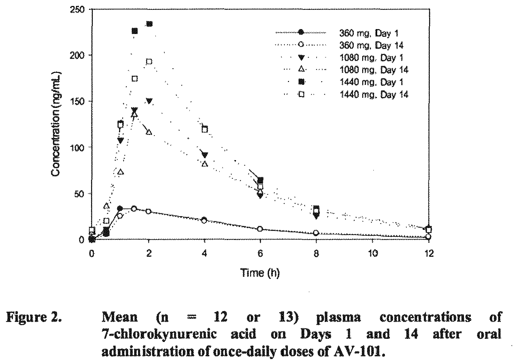

A pharmaceutical composition of the invention may be formulated in any pharmaceutical form that contains L-4-chlorokynurenine according to the invention and that produces a blood plasma level of 7-chlorokynurenic acid as described in this application. It is contemplated that the exact dosages of L-4-chlorokynurenine to be administered within the ranges described for the present invention are to be safe and effective, and that they produce plasma levels of 7-chlorokynurenic acid resulting from the administration of L-4-chlorokynurenine as described in this patent application at Fig. 2 and in other places. Thus, plasma ranges of 7-chlorokynurenic acid from about 15 ng/mL to about 65 ng/m, from about 65 ng/mL to about 300 ng/mL and from about 300 ng/mL to about 550 ng/mL are expressly contemplated. It is also contemplated that the unit dose formulation of the present invention may be administered one or more times per day, and over multiple days, such a two, three or four times weekly or every other day, in order to extend the time period in which 4-chlorokynurenine levels are elevated to a therapeutically effective amount of 7-chlorokynurenic acid.

The unit dose pharmaceutical compositions of L-4-chlorokynurenine for oral administration for the treatment of certain indications, such as treatment of pain, as discussed in this specification preferably contain about 50 mg to about 1,800 mg, more preferably about 260 mg to about 1,540 mg, more preferably either about 260 mg to about 460 mg, about 310 mg to about 410 mg, about 460 mg to about 980 mg, about 980 mg to about 1,180 mg, about 1,030 mg to about 1,130 mg, about 1,340 to about 1,540 mg, about 1,390 mg to about 1,490 mg and most preferably about 360, 1,080 or 1,440 mg. For other indications, such as depression, the foregoing dose is appropriate, although a precise dose may be determined by the health care provider by initiating treatment at an appropriate dose, for example, within the dose ranges described above or as otherwise may be determined, and then monitoring the health and symptoms of the treated patient to permit adjustment of the dose by small increments or as determined. Preferably, the patient will be reevaluated periodically to determine the improvement and clinical benefit of the administration of L-4-chlorokynurenine. Generally, the dose may be administered in a single dose or as multiple doses at intervals as may be appropriate, for example in two, three or four sub-doses per day to achieve a therapeutically effective amount of L-4-chlorokynurenine.

It is contemplated that the dosing regimens for the compositions of the present invention are therapeutically effective. While a daily dosing regimen is contemplated, as described above, this would preferably be from about 5 to about 30 days, including shorter and longer dosing regimes as determined by a patient's physician. In particular, dosing regimes of about 7 to about 24 days, and about 12 to about 16 days are expressly contemplated.

A preferred aspect of the present invention involves the administration of 4-chlorokynurenine in conjunction with L-DOPA to reduce the dyskinesia associated with maintenance doses (typically, determined empirically for each patient by the physician) of L-DOPA; or to reduce the minimum effective dose of L-DOPA, thereby delaying the onset and/or reducing the severity of the dyskinesia. Pharmaceutical compositions according to the present invention may be administered in combination with L-DOPA, concurrently or closely enough in temporal proximity to ameliorate the side effects of L-DOPA administration. Protocols for the administration of L-DOPA are well known, as are associated dyskinesias. See, for example, Tambasco et al. (2012).□

The pharmaceutical composition according to the present invention may be, for example, a tablet, capsule, liquid suspension, solid solution, softgel, injectable, topical, or transdermal, or suppository and nasal delivery. Additionally, a pharmaceutical composition of the present invention can also be a modified release form such as, but not limited to, a bi-modal or extended release form.

In general, the pharmaceutical compositions of the invention may be prepared by conventional methods know in the art of pharmaceutical formulations. For example, see Remington's Pharmaceutical Sciences, 18th Ed., (Mack Publishing Company, Easton, Pa., 1990), which is incorporated herein by reference. In a solid dosage form, L-4-chlorokynurenine may be admixed with at least one pharmaceutically acceptable excipient such as, for example, sodium citrate or dicalcium phosphate or (a) fillers or extenders, such as, for example, starches, lactose, sucrose, glucose, mannitol, and silicic acid, (b) binders, such as, for example, cellulose derivatives, starch, alignates, gelatin, polyvinylpyrrolidone, sucrose, and gum acacia, (c) humectants, such as, for example, glycerol, (d) disintegrating agents, such as, for example, agar-agar, calcium carbonate, potato or tapioca starch, alginic acid, croscarmellose sodium, complex silicates, and sodium carbonate, (e) solution retarders, such as, for example, paraffin, (f) absorption accelerators, such as, for example, quaternary□ammonium compounds, (g) wetting agents, such as, for example, cetyl alcohol, and glycerol monostearate, magnesium stearate and the like (h) adsorbents, such as, for example, kaolin and bentonite, and (i) lubricants, such as, for example, talc, calcium stearate, magnesium stearate, solid polyethylene glycols, sodium lauryl sulfate, or mixtures thereof. In the case of capsules, tablets, and pills, the dosage forms may also comprise buffering agents.

Pharmaceutically acceptable adjuvants known in the pharmaceutical formulation art may also be used in the pharmaceutical compositions of the invention. These include, but are not limited to, preserving, wetting, suspending, sweetening, flavoring, perfuming, emulsifying, and dispensing agents. Prevention of the action of microorganisms may be ensured by inclusion of various antibacterial and antifungal agents, for example, parabens, chlorobutanol, phenol, sorbic acid, and the like. It may also be desirable to include isotonic agents, for example, sugars, sodium chloride, and the like. If desired, a pharmaceutical composition of the invention may also contain minor amounts of auxiliary substances such as wetting or emulsifying agents, pH buffering agents, antioxidants, and the like, such as, for example, citric acid, sorbitan monolaurate, triethanolamine oleate, butylated hydroxytoluene, etc.

Solid dosage forms as described above may be prepared with coatings and shells, such as enteric coatings and others well known in the art. They may contain pacifying agents, and can also be of such composition that they release the active compound or compounds in a certain part of the intestinal tract in a delayed manner. Non-limiting examples of embedded compositions that may be used are polymeric substances and waxes. The active compounds may also be in microencapsulated form, if appropriate, with one or more of the above-mentioned excipients.

Suspensions, in addition to the active compounds, may contain suspending agents, such as, for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.□

Compositions for rectal administrations are, for example, suppositories that may be prepared by mixing the crystalline genistein sodium salt dihydrate according to the invention with, for example, suitable non-irritating excipients or carriers such as cocoa butter, polyethylene glycol or a suppository wax, which may be solid at ordinary temperatures but may be liquid at body temperature and, therefore, melt while in a suitable body cavity and release the active component therein.

### Clinical Study:

A Phase lb, single-site, randomized, double-blind, placebo-controlled, study was conducted involving multiple oral doses of L-4-chlorokynurenine in healthy male and female subjects. Subjects were randomized into one of three cohorts (360, 1,080, and 1,440 mg) and received a daily oral dose for 14 consecutive days. Each cohort originally was planned to include 12 subjects on active drug and 4 subjects on placebo. However, a total of 50 subjects were enrolled in this study. In Cohort 1, 12 subjects received L-4-chlorokynurenine and 5 subjects received placebo. In Cohort 2, 13 subjects received L-4-chlorokynurenine and 4 subjects received placebo. In Cohort 3, 12 subjects received L-4-chlorokynurenine and 4 subjects received placebo. Forty-six subjects completed the study per protocol. The safety, pharmacokinetics (PK), tolerability of treatment, and antihyperalgesic effect of L-4-chlorokynurenine on capsaicin-induced hyperalgesia were assessed.

The following PK parameters were derived from the plasma concentration versus time profiles to determine the single-dose and multiple-dose PK profile of L-4-chlorokynurenine and the active metabolite, 7-chlorokynurenic acid: Maximum concentration (Cₘₐₓ), Terminal elimination half-life (t_{½}), Time to maximum concentration (Tₘₐₓ), Area under the plasma study drug concentration versus time curve from Time 0 to time of last measurable concentration (AIC₀₋ₜ) and Area under the plasma study drug concentration versus time curve from Time 0 extrapolated to infinity (AIC_{0-∞}).□

Blood was collected in 6-mL lithium heparin Vacutainer tubes. Samples were centrifuged, and the plasma was separated from the cells and frozen within 30 minutes after centrifugation. Centrifuged samples were placed on ice until they were placed in the freezer. Plasma samples were stored frozen at approximately -20°C until their shipment to a contractor for analysis.

Baseline samples (0 minutes) were collected before the drug was administered on Day 1 and on Day 14. Samples were then collected at 0.5, 1, 1.5, 2, 4, 6, 8, 12, and 24 hours after dosing on Day 1 and Day 14.

A liquid chromatography with tandem mass spectrometry assay was used for the determination of 7-chlorokynurenic acid and L-4-chlorokynurenine in human plasma. The standard curve range was from 2.00 to 1,000 ng/mL for 7-chlorokynurenic acid, with a lower limit of quantification of 2.00 ng/mL. The standard curve range was from 0.05 to 50 µg/mL for L-4-Cl-KYN with a lower limit of quantification of 0.05 µg/mL. Both assays used a plasma sample volume of 50.0 µg/mL.

The PK of L-4-chlorokynurenine was fully characterized across the range of doses in the study. Plasma concentration-time profiles obtained for L-4-chlorokynurenine and 7-chlorokynurenic acid after administration of a single and multiple, once daily oral doses of 360, 1,080, or 1,440 mg were consistent with rapid absorption of the oral dose and first-order elimination of both analytes, with evidence of multicompartment kinetics, particularly for the metabolite 7-chlorokynurenic acid. Mean L-4-chlorokynurenine Tmax values increased with increasing dose level, reaching nearly 2 hours for the highest dose group. Mean t_{½} values were fairly consistent across doses, ranging from 1.64 to 1.82 hours. Mean L-4-chlorokynurenine Cₘₐₓ and AIC_{0-∞} values appeared to be approximately dose linear, although not proportional. Mean Cₘₐₓ values on Day 1 ranged from 27.7 µg/mL after the 360-mg dose to 64.4 µg/mL after the 1,440-mg dose. Mean AIC₀₋ₜ values on Day 1 ranged from 64 µg·h/mL after the lowest dose to 196 µg·h/mL after the 1,440-mg dose. Mean Cₘₐₓ and AIC₀₋ₜ values the majority of the times were slightly lower on Day 14 than those on Day 1.

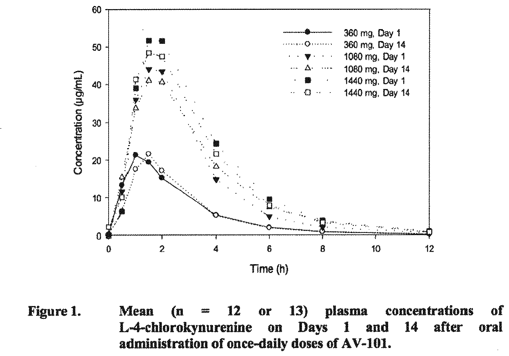

In general, as expected for a metabolite, 7-chlorokynurenic acid maximum concentrations occurred at the same time or later than those for L-4-chlorokynurenine, with mean Tₘₐₓ values ranging from 1.67 to 2.34 hours. This is shown in Figs. 1 and 2. Mean t_{½} values for 7-chlorokynurenic acid, ranging from 2.52 to 3.23 hours, were slightly more variable across doses than those for L-4-chlorokynurenine. Mean t_{½} values did not appear to be dose related. Mean 7-chlorokynurenic acid Cₘₐₓ and AIC₀₋ₜ values also appeared to be approximately dose linear. Mean Cₘₐₓ values on Day 1 ranged from 42.7 ng/mL after the 360-mg dose to 314 ng/mL after the 1,440-mg dose. Mean AIC₀₋ₜ values on Day 1 ranged from 156 ng-h/mL after the lowest dose to 985 ng-h/mL after the 1,440-mg dose. Similar to the parent compound, mean Cₘₐₓ and AIC₀₋ₜ values for 7-chlorokynurenic acid generally were slightly lower on Day 14 than those on Day 1.

### EXAMPLE 1: Antihyperalgesic effect of L-4-chlorokynurenine on capsaicin-induced hyperalgesia.

On Day 1 and Day 14 of the clinical study, two intradermal injections of 250 µg of capsaicin were injected sequentially into the volar aspect of alternate forearms to produce burning pain, secondary hyperalgesia, and a flare. The capsaicin USP (United States Pharmacopeia) was prepared according to the site's standard procedure and dissolved in 20% cyclodextrin at a concentration of 10 mg/mL.

The first capsaicin injection in one forearm was given 1 hour after oral administration of L-4-chlorokynurenine or placebo, and the second capsaicin injection was given in the other forearm 2 hours after the administration of L-4-chlorokynurenine or placebo. The neurosensory testing began immediately at each capsaicin injection. The serial pain assessments using a 100-mm visual analog scale (VAS) occurred at preinjection and 0, 5, 10, 15, 30, 45, and 60 minutes after each capsaicin injection. The examiner asked the subject to rate the intensity by using the VAS of the spontaneous pain and elicited pain from the application of a 5.18 von Frey hair, 40°C probe, and gentle stroking with a 1-inch foam brush. The VAS consisted of a 100-mm line with "no pain" written at the 0-mm end and the "worst imaginable pain" written at the 100-mm end. The distance in millimeters provided the pain measurement.

The borders of the hyperalgesic area to a 5.18 von Frey hair were determined by moving from an area of the skin that did not produce pain tangentially toward the center of the painful area at a progressively closer radius until the subject reported pain or tenderness. At least eight determinations of the hyperalgesic area borders were made using the same assessments and starting at different angles. Additionally, subjects were asked to rate the pain intensity of a 1-minute 45°C heat stimulus (brief thermal stimulation) applied to the anterior thigh at 4.5 hours (±5 minutes) after study drug administration, then every 30 minutes (±5 minutes) through 6 hours after study drug administration. A radiant temperature probe was applied to fix the skin temperature at 36°C during the pain assessments. Subjects used the VAS for all pain assessments.

All study assessment time points were anchored to Time 0, the time of study drug administration. For the capsaicin injection at 1 hour after dosing, the assessment interval (i.e., time after dosing of the study drug) was 60 to 120 minutes, during which time assessments occurred at approximately 60, 65, 70, and 75 minutes and then every 15 minutes through the 120-minute time point (i.e., 0, 5, 10, 15, 30, 45, and 60 minutes after capsaicin injection). The second injection of capsaicin was administered approximately 2 hours after dosing of the clinical trial material (CTM), and the assessment interval (i.e., time after dosing of the CTM) was 120 to 180 minutes. The serial pain assessments followed the same schedule, as described after the first capsaicin injection.

The primary efficacy endpoint was the analgesic response to spontaneous pain at each dose level of L-4-chlorokynurenine 120 to 180 minutes after dosing on Day 14. There was no significant change in the area under the pain time curve (AUPC) for the spontaneous pain assessment between the treatment and the placebo groups. Likewise, there were no significant changes between the treatment and the placebo groups for any of the secondary efficacy endpoints (AUPC for spontaneous pain for the time interval of 120 to 180 minutes after dosing on Day 1; and AUPC for spontaneous pain, elicited pain from the von Frey hair, and elicited pain from the 40°C probe for the time interval of 60 to 180 minutes after dosing on Days 1 and 14). However, the present inventors found a consistent decrease in the least squares means of the AUPC for allodynia pain, mechanical hyperalgesia pain, and heat hyperalgesia pain between subjects that received 1,080 mg L-4-chlorokynurenine (Cohort 2) and subjects that received placebo. These data are shown in Table 1.

**Table 1: Pain evaluation scores**

| **Heat Allogesia** | | | |
|---|---|---|---|
| | n | MAXPAIN | MP StdDiv |
| placebo | 4 | 84.75 | 5.74 |
| 1080 mg | 13 | 78.85 | 16.22 |

| **Mechanical Allogesia** | | | |
|---|---|---|---|
| | n | MAXPAIN | MP StdDiv |
| placebo | 4 | 79.75 | 7.93 |
| 1080 mg | 13 | 66.23 | 25.60 |

| **Allodynia Pain (foam brush)** | | | |
|---|---|---|---|
| | n | MAXPAIN | MP StdDiv |
| placebo | 4 | 79.50 | 9.68 |
| 1080 mg | 13 | 70.92 | 21.63 |

| **Spontaneous Pain** | | | |
|---|---|---|---|
| | n | MAXPAIN | MP StdDiv |
| placebo | 4 | 77.00 | 10.23 |
| 1080 mg | 13 | 75.08 | 23.33 |

### Treatment of Depression:

The Centers for Disease Control and Prevention estimate that about 10% of US adults meet the criteria for "current depression," defined as being either "major depression" or "other depression." Major depressive disorder ("MDD") is associated with severe morbidity and mortality, contributing to suicide, the presence of mental illness and its adverse outcomes, interference with interpersonal relationships, substance abuse, and time lost from work. And recurrent mood disorders such as MDD and bipolar disorder (BPD) are chronic and often life threatening. For example, suicide is estimated to be the cause of death in up to about 15% of individuals with MDD. Additionally, depression produces adverse effects on other disease conditions. For example, see Musselman et al. 1988.

A variety of depression conditions and mood disorders may be treated with an effective amount of L-4-chlorokynurenine according to the present invention without unduly affecting behavior or motor coordination, and without inducing or promoting seizure activity. A physician or psychologist, for example, may ascertain symptoms of depression, and its relief, by a mental state examination using known approaches including the various scales discussed in this specification. Symptoms of such depression conditions and mood disorders include thoughts of hopelessness, self-harm or suicide and/or an absence of positive thoughts or plans. Such conditions and disorders include major depressive disorder (MDD), dysthymic disorder (or dysthymia), persistent depressive disorders, atypical depression, bipolar depression or manic depression, seasonal affective disorder (SAD), psychotic depression and postpartum depression, psychotic depression, premenstrual syndrome, premenstrual dysphoric disorder, anxiety, mood disorder, depressions caused by chronic medical conditions such as cancer or chronic pain, chemotherapy, chronic stress, post-traumatic stress disorders, risk of suicide. It should be understood that depression caused by bipolar disorder might also be referred to as bipolar depression. In addition, patients suffering from any form of depression often experience anxiety. Various symptoms associated with anxiety include fear, panic, heart palpitations, shortness of breath, fatigue, nausea, and headaches among others. It is expected that the methods of the present condition can be used to treat anxiety or any of the symptoms thereof.

In addition, a variety of other neurological conditions may be treated according to the methods of the invention. Exemplary conditions include, but are not limited to obsessive-compulsive disorder, learning disorder, autistic disorder, attention-deficit hyperactivity disorder, Tourette's syndrome, phobia, post-traumatic stress disorder, dementia, AIDS dementia, Alzheimer's disease, Parkinson's disease, Huntington's disease, spasticity, myoclonus, muscle spasm, bipolar disorder, a substance abuse disorder, urinary incontinence, and schizophrenia. Also provided herein are methods of treating depression in treatment resistant patients or treating refractory depression, that is patients suffering from a depression disorder that does not, and/or has not, responded to adequate courses of at least one, or at least two, other antidepressant compounds or therapeutics by the administration of an effective amount of L-4-chlorokynurenine as described in this specification.

Without being held to a particular mechanism of action, it is believed that because L-4-chlorokynurenine targets, that is to say it blocks or antagonizes the glycine co-agonist site of the receptor, its therapeutic administration may avoid causing the potential psychotomimetic side effects that occur with ketamine, as determined by the drug discrimination, conditioned place preference, and pre-pulse inhibition tests, without affecting the efficacy of L-4-chlorokynurenine. This may then result in the "glutamate surge" resulting in an AMPA receptor-dependent synaptogenesis that has been associated with the rapid acting antidepressant effects of ketamine.

It is also contemplated that L-4-chlorokynurenine is effective to treat suicidal patients in acute emergency situations such as in a hospital emergency room. For this use of the present invention, administration of L-4-chlorokynurenine as an injectable or suppository formulation is preferred for those patients that are not able to take oral administration. Appropriate formulations are known to persons skilled in the art and are described above or incorporated by reference to various documents discussed or cited in this specification.

The preferred dose range for the treatment of depression is from about 20 mg/day up to about 2,000 mg/day, more preferably from about 300 mg/day to about 1,500 mg/day and more preferably from about 700 mg/day to about 1,200 mg/day. Within these preferred dose ranges, Applicant contemplates that 340 mg/day, 1,080 mg/day and 1,440 mg/day also are preferred.

### EXAMPLE 2: Anti-Depressive Activity of L-4-chlorokynurenine

The present inventors surprisingly also found a mood enhancing or anti-depressive activity of L-4-chlorokynurenine. In the clinical study described in this application, 5 out of 26 subjects (as contrasted with zero subjects in the placebo group) affirmatively reported feelings of well being. This is consistent with reports that the glutamatergic system contributes to the pathophysiology of depression and that stress can induce changes in NMDA receptors. See, for example, Calabrese et al. 2012.

### EXAMPLE 3: Treatment of Major Depressive Disorder (MDD)

Twenty-five patients, both male and female, ages 18 to 65, with a diagnosis of MDD are treated with either L-4-chlorokynurenine (1,080 or 1,440 mg/day given orally) for 2 weeks, similar in design to similar studies. [Ibrahim et al. 2012, Zarate et al. 2013, Zarate et al. 2006, Zarate et al. 2005.] Improvement in overall depressive symptomatology is shown by a significant decrease in either or both of the Hamilton Depression Rating Scale (HDRS) [Hamilton 1959] and the Montgomery Asberg Depression Rating Scale (MADRS) total scores. [Montgomery et al. 1979] Additional indicators of therapeutic efficacy for a given patient could also be the proportion of subjects achieving remission (HDRS ≤7) and response (≥50% reduction from baseline in HDRS total score); change from baseline in Hamilton Anxiety Rating Scale (HAM-A) [Hamilton 1959], Columbia Suicide Severity Rating Scale (C-SSRS) total scores [Posner et al. 2011], as well as other measures of mood or psychological states, for example Beck Depression Inventory (BDI) [Beck et al., 1974], the Visual Analogue Scale (VAS) [Aitken 1969], the Brief Psychiatric Rating Scale (BPRS) [Overall et al. 1962], the Clinician Administered Dissociative Scale (CADSS) [Bremner et al. 1998] and the Young Mania Rating Scale (YMRS) [Young et al. 1978].

### Combination Therapy for Treatment of Depression:

L-4-chlorokynurenine may be administered in combination with other agents useful in treating depression and other conditions described in this specification, and such combinations may be synergistically effective when so administered. A synergistic response with the two therapies achieves a better outcome in a given patient, in terms of extent, duration or reduction in side effects, control of the symptoms and, for example, ideation associated with depression such as MDD.

Appropriate therapeutically active agents for co-administrations will be identifiable by skilled practitioners, and will be administered in the typical dosage ranges known to and used by clinicians. The dosage of L-4-chlorokynurenine may be used as described in this specification. With regard to depression, the agent that is co-administered is typically the clinicians' choice of antidepressant therapy (ADT) which results in: 1) an inadequate response to ADT, or 2) an adequate response to ADT but producing unacceptable side effects. In the first case, L-4-chlorokynurenine is given with the ADT at an approved dose. In the second case, L-4-chlorokynurenine is given with the ADT at an approved dose that is reduced sufficiently to reduce the negative side effects to an acceptable level. Persons skilled in the art would know to administer the ADT within the approved range and, where side effects are a concern, would decrease the dosage of that ADT while monitoring a patient's response.

Appropriate agents that are contemplated for combination administration for the treatment of depression include:
- Glutamate-modulating agents, such as Riluzole, Lamotrigine, Topiramate, Pregabalin, Acamprosate, Aniracetam, EMQMCM, MTEP, LY341495, RO4491533, ACPT-1, AMN082, RS-PPG
- Other NMDA regulating compounds, such as Memantine, Lanicemine, GLYX-13, NRX-1074, Traxoprodil, Selfotel, Cerestat, dextro-methorphan, besonprodil, Ro25-6981
- Selective serotonin reuptake inhibitors (SSRIs), such as Citalopram (Celexa), Escitalopram (Lexapro, Cipralex), Paroxetine (Paxil, Seroxat) Fluoxetine (Prozac), Fluvoxamine (Luvox), and Sertraline (Zoloft, Lustral)
- Serotonin-norepinephrine reuptake inhibitors (SNRIs), such as Desvenlafaxine (Pristiq), Duloxetine (Cymbalta), Levomilnacipran (Fetzima), Milnacipran (Ixel, Savella), Tofenacin (Elamol, Tofacine), and Venlafaxine (Effexor)
- Serotonin modulators and stimulators (SMSs), such as Vilazodone (Viibryd), Vortioxetine (Brintellix)
- Serotonin antagonists and reuptake inhibitors (SARIs), such as Etoperidone (Axiomin, Etonin), and Trazodone (Desyrel)
- Norepinephrine reuptake inhibitors (NRIs), such as Reboxetine (Edronax), Viloxazine (Vivalan), and Atomoxetine (Strattera)
- Serotonin reuptake enhancer (SSRE), such as Tianeptine (Stablon)
- Tricyclic antidepressants (TCAs), such as Amitriptyline (Elavil, Endep),
- Butriptyline (Evadene), Clomipramine (Anafranil), Desipramine (Norpramin, Pertofrane), Dosulepin (Prothiaden), Doxepin (Adapin, Sinequan), Imipramine (Tofranil), Iprindole (Prondol), Lofepramine (Feprapax, Gamanil, Lomont),
- Melitracen (Melixeran), Nortriptyline (Pamelor), Protriptyline (Vivactil), and
- Trimipramine (Surmontil)
- Sigma receptor agonists, such as Opipramol (Insidon)
- Tetracyclic antidepressants (TeCAs), such as Amoxapine (Asendin), Maprotiline (Ludiomil), Mianserin (Bolvidon, Norval, Tolvon), Mirtazapine (Remeron), Setiptiline (Tecipul)
- Noradrenergic and specific serotonergic antidepressants (NaSSAs), such as Mianserin, mirtazapine, and setiptiline are also sometimes described as tetracyclic antidepressants.
- Monoamine oxidase inhibitors (MAOIs), such as Isocarboxazid (Marplan), Phenelzine (Nardil), Tranylcypromine (Parnate), Selegiline (Eldepryl, Zelapar, Emsam), Metralindole (Inkazan), Moclobemide (Aurorix, Manerix), Pirlindole (Pirazidol), Toloxatone (Humoryl)
- Agomelatine (Valdoxan) - 5-HT2C receptor antagonist and MT1 and MT2 receptor agonist
- Buprenorphine (Subutex, Temgesic, Buprenex) - κ-opioid receptor antagonist and µ-opioid receptor partial agonist
- Bupropion (Wellbutrin) - NRI and non-competitive antagonist of several neuronal nACh receptors
- Tandospirone (Sediel) - 5-HT1A receptor partial agonist
- Teniloxazine (Lucelan, Metatone) - NRI and 5-HT2A receptor antagonist

Other adjunctive treatments are also appropriate for co-administration with L-4-chlorokynurenine. This category includes drugs that are generally not considered to be significantly effective as treatments for depression alone, but have demonstrated effectiveness in the augmentation of antidepressant efficacy when co-administered. Such drugs include:
- Atypical antipsychotics, such as Aripiprazole (Abilify), Lurasidone (Latuda), Olanzapine (Zyprexa), Quetiapine (Seroquel, Seroquel XR), Risperidone (Risperdal), Ziprasidone (Geodon)
- Others, such as, Buspirone (BuSpar) - 5-HT1A receptor partial agonist; Lithium (Eskalith, Lithobid) - mood stabilizer; Thyroxine (T4) - thyroid hormone; Triiodothyronine (T3) - thyroid hormone
- Current combination products
- Olanzapine/fluoxetine (Symbyax) - SSRI and atypical antipsychotic combination
- Herbal medicines, such as St John's Wort

It is also contemplated that co-administration of L-4-chlorokynurenine with known therapeutic agents would be appropriate for the treatment of other conditions discussed in this specification, including, for example, obsessive-compulsive disorder and bipolar disorder.

### EXAMPLE 4: Combination Therapy with ADT and L-4-chlorokynurenine

Patients who experience a major depressive episode and receive their clinicians' choice of antidepressant therapy (ADT) sometimes have an inadequate therapeutic response to that agent and are given another ADT in combination with the first [Hori et al 2012, Kamijima et al. 2013, Macfadden et al. 2011, Quante et al. 2013, Sepanjnia 2012]. L-4-chlorokynurenine together with another ADT (as discussed above), is administered to such patients. Improvement in extent or duration of symptoms are shown, as determined by one or more of the above measures that exceed the effects seen by either drug alone, similar to the synergistic response known to persons skilled in the art when administering sub-therapeutic doses of ketamine and alpha-amino-3-hydroxy-5-methyl-4-isoxazolepropionic acid (AMPA) [Akinfiresoye et al. 2013]. The dose of L-4-chlorokynurenine is selected from about 20 mg/day up to about 2,000 mg/day, more preferably about 300 mg/day to about 1,500 mg/day and more preferably about 700 mg/day to about 1,200 mg/day.

### Cerebrospinal Fluid Biomarkers for L-4-chlorokynurenine Administration:

A "biomarker" or "moderator" as elaborated by Kraemer et al. 2002, are factors that "specify for whom and under which conditions the treatment works. They also suggest to clinicians which of their patients might be most responsive to the treatment and for which patients other, more appropriate, treatments might be sought". There is precedent for several types of biomarkers being used to predict and follow therapy for depression and other psychiatric diseases and conditions. [Hunter et al. 2011, Ising et al. 2007, Ji et al. 2011, Siegle et al. 2012, Wolkowitz et al. 2012.]

Applicants surprisingly have discovered that the measurement of cerebrospinal fluid (CSF) levels of kynurenic acid (KYNA) and quinolinic acid (QUIN) is useful to stratify patients for the probability of beneficial drug response to L-4-chlorokynurenine. Consistent with this finding is the known association between low KYNA and high QUIN in suicidal patients. [Bay-Richter et al. 2015, Erhardt et al. 2013, Myint et al. 2007]

L-4-chlorokynurenine therapeutic activity is positively correlated in patients with levels of KYNA in the CSF. The medical literature reports a significant association between low kynurenic acid and severe depressive symptoms. Because L-4-chlorokynurenine is metabolized into 7-Cl-KYNA, which is about 20x more potent and selective than the endogenous naturally occurring KYNA, L-4-chlorokynurenine will help to normalize KYNA functional levels to moderate and normalize NMDA receptor activity. In addition, L-4-chlorokynurenine may help to reduce the pathological, and in some cases neurotoxic, levels of QUIN, due to the fact that one of its metabolites, 4-Cl-3-hydroxyanthranilic acid, inhibits the synthesis of quinolinic acid.

QUIN is a naturally occurring NMDA receptor agonist that causes convulsions and excitotoxic damage at pathological levels. Therefore, because L-4-chlorokynurenine will decrease CSF levels of quinolinic acid, its therapeutic activity will be positively correlated in patients who present with elevated levels of QUIN. And those patients who present with the highest levels of quinolinic acid will experience the largest reduction in quinolinic acid in the CSF and typically exhibit a relatively larger therapeutic response.

It is contemplated that symptomatic patients with levels of KYNA determined to be in the bottom three quartiles are likely to and would be expected to benefit most and show greatest clinical improvement from administration of L-4-chlorokynurenine to treat MDD and other forms of depression; similarly, responses and improvements are expected for those patients in the bottom two quartiles; and responses and improvements are expected particularly for those patients in the bottom quartile. Conversely, it is contemplated that symptomatic patients with levels of QUIN determined to be in the top two quartiles are likely to and would be expected to benefit and show clinical improvement from administration of L-4-chlorokynurenine to treat MDD and other forms of depression; similarly, responses and improvements are expected for those patients in the top two quartiles; and responses and improvements particularly are expected for those patients in the top quartile.

It is contemplated that symptomatic patients with low levels of KYNA and high levels of QUIN would be would be expected to benefit most, and show maximal clinical improvement from administration of L-4-chlorokynurenine to treat MDD and other forms of CNS-related diseases or conditions. Accordingly, a personalized medicine diagnostic test for low levels of KYNA and/or high levels of QUIN is contemplated, preferably based on blood levels of such compounds.

### Treatment of Obsessive-Compulsive Disorder:

Patients with obsessive-compulsive disorder (OCD) are treated with L-4-chlorokynurenine to reduce OCD symptoms. Patients show improvement based on assessment with assessment tests, such as the OCD visual analog scale (OCD-VAS) and the Yale-Brown Obsessive-Compulsive Scale (Y-BOCS). The dose of L-4-chlorokynurenine is selected from about 20 mg/day up to about 2,000 mg/day, more preferably about 300 mg/day to about 1,500 mg/day and more preferably about 700 mg/day to about 1,200 mg/day.

### Treatment of Tinnitus:

Tinnitus, commonly described as a ringing in the ears, is the perception of sound in the absence of an auditory stimulus. Approximately 1 out of 10 adults in the United States has experienced tinnitus lasting at least five minutes in the past year, for at least 1 in 100, tinnitus severely affects their quality of life. This amounts to over 13 million people in Western Europe and the USA with serious tinnitus. [Vio et al. 2005, Axelsson et al. 1989] Often, severe tinnitus is associated with depression, anxiety and insomnia. [Langguth et al. 2007, Cronlein et al. 2007] The "US Veterans Administration 2013 Benefits Report" ranked tinnitus as one of the top most prevalent service-connected disabilities of new Veterans, accounting for 9.5% of all claims (http://www.benefits.va.gov/REPORTS/abr/ABR-Combined-FY13-09262014.pdf).

Despite the significant clinical need for effective treatment of tinnitus, there is currently no single FDA- or EMEA-approved drug for tinnitus. Thus, another aspect of the present invention relates to the use of L-4-chlorokynurenine in treating tinnitus, through its administration at dosage ranges described in this specification. Types of tinnitus for which this therapy is contemplated include subjective tinnitus; noise-induced hearing loss; hearing loss associated with drug or chemical effects, especially ototoxic drugs such as gentamicin, furosemide and platinum-based chemotherapy agents such as cisplatin, nonsteroidal antiinflammatory drugs (NSAIDS), and mercury or lead poisoning; hearing loss associated with other medical conditions, for example, Ménière's disease, acoustic neuroma, vestibular schwannoma, glomus tympanicum tumor, fibromyalgia, migraine, vasculitis, atherosclerosis; hearing loss associated with neurological damage, for example, multiple sclerosis, ear infections, oxidative and emotional stress, encephalitis, cerebrospinal fluid leak; and hearing loss associated with cerebral head injury or malformations, for example, Arnold-Chiari malformation.

Without being held to a particular mechanism of action for this aspect of the present invention, it has been proposed that NMDA receptor-mediated excitotoxicity is a mechanism for cochlear tinnitus [Guitton et al. 2003, Guitton et al. 2007, Oestreicher et al. 1998]. NMDA receptor-mediated pathways are associated with salicylate-induced tinnitus [Peng et al. 2003], and NMDA antagonists applied locally to the inner ear blocked behavioral evidence of salicylate-induced tinnitus. [Guitton et al. 2003] Furthermore, cochlear application of a selective NMDA antagonist in the first four days following noise exposure also reduced the probability of developing noise-induced tinnitus. [Guitton et al. 2007]

### EXAMPLE 5: Treatment of Tinnitus

A clinical study to demonstrate the efficacy of L-4-chlorokynurenine in the treatment of tinnitus is a prospective, randomized, double blind, placebo-controlled, parallel group dose-escalation design. One hundred male or female patients between 20-65 years of age with mon- or binaural chronic tinnitus lasting for at least 3-months are enrolled in the study. Patients are randomized into a drug and placebo groups, baseline psychometric and health measurements are taken, followed by blind administration of a daily single oral dose for 14-days of either L-4-chlorokynurenine (initial dose of 180 mg) or placebo. Subsequent psychometric and health measurements are taken at 3-, 7-, and 14-days post (prior to next drug administration) drug administration. At 14, and 28 days the dose of AV-101 is increased to 720 and 1440 mg/day respectively. At 42 and 70 days, all patients are given the panel of psychometric and health measurements.

Treated subjects show improvement in the symptoms of tinnitus based on psychometric data derived from two established self-evaluation instruments: 1) a standardized tinnitus questionnaire (TQ) based on Hallam's TQ [Hallam 2007], and the Short Form 36 health survey (SF-36). The SF-36 questionnaire is a well-established assessment tool for is a well as accepted measure for the observation of clinical course and therapy control in tinnitus. [Nondahl et al. 2007, Van Hook et al. 1996]

In addition to standard safety and tolerability assessment typical of a Phase 2 study, subjects also show improvement when objective audiometric parameters are assessed, such as a pure tone audiogram, and measurements to assess tinnitus loudness. [Tyler et al. 1983]

Primary success measures are a statistically significant difference between the mean TQ scores of L-4-chlorokynurenine and placebo groups. Secondary measures include significant changes in SF-36 or audiometric data. Student t□test for independent samples or the Mann-Whitney test at each time point are used to assess the statistical significance of the quantitative data. The significance of the qualitative data is assessed with chi-square test or the Fisher exact test. To assess improvement over time and dose, TQ and secondary measurements are evaluated using Friedman Variance Analysis and similar tools for variance analysis.

### Treatment of Diabetes:

As of 2014, an estimated 387 million people have diabetes worldwide [WHO 2013], with type 2 diabetes (T2DM) making up about 90% of the cases. [Shi et al, 2014] This represents 8.3% of the adult population [Shi et al., 2014] with equal rates in both women and men. [Vos et al. 2012] From 2012 to 2014, diabetes is estimated to have resulted in 1.5 to 4.9 million deaths each year. [WHO 2013, Vos et al. 2012] Diabetes at least doubles a person's risk of death. [WHO 2015] The number of people with diabetes is expected to rise to 592 million by 2035. [WHO 2013]

The global economic cost of diabetes in 2014 was estimated to be $612 billion USD. [IDF 2013] In the United States, diabetes cost $245 billion in 2012. [Yang et al. 2013] Many of the existing drugs, for example, sulfonylureas used to treat T2DM not only increase glucose-stimulated insulin secretion (GSIS), but also basal insulin secretion. This increase in basal insulin secretion by these drugs can result in hypoglycemia that can be medically serious and can lead to death. [Amiel et al. 2008] Therefore, there is a significant need for safer drugs that increase GSIS without increasing basal insulin secretion.

Diabetes results from a reduction in the production of insulin (Type 1), or a poor response, or resistance, to insulin by the cells of the body (Type 2). Several studies support the role that neurons play, together with β islet cells, to regulate insulin and glucose levels. [Schwartz et al. 2013] Although, neurons can produce insulin [Kuwabara et al. 2011], pancreatic β islets are responsible for the major production of insulin. Interestingly, β islet cells are enervated [Burris et al. 2007] and share many receptors and molecular pathways in common with neurons. [Soltani et al. 2011, Rodriguez-Diaz et al. 2013] For example, β islet cells express several components of the glutamate signaling system, including excitatory amino acid transporters, AMPA and receptors, glutamate transporters, and NMDA receptors. [Vetterli et al. 2012, Inagaki et al. 1995, Marquard et al. 2015]

It is contemplated that patients diagnosed with T2DM be treated with L-4-chlorokynurenine, administered orally with a daily dose in the range of 20 to 1,440 mg, more specifically at 20, 180, 360, 720, 1,080 or 1,440 mg either as a monotherapy, or an adjunctive therapy with metformin similar to other diabetes studies. [Rosenstock et al. 2014, Group 2013, Skrivanek et al. 2014] Such doses of L-4-chlorokynurenine optionally may be combined with incretin-based drugs for treating diabetes, for example, DPP-4 inhibitors and GLP-1 analogs. [Tasyurek et al. 2014, Hainer 2014] Other types of diabetes contemplated for treatment with the methods and compositions of the present invention include Type 1 diabetes, insulin-dependent diabetes, juvenile diabetes and early-onset diabetes; Type 2 diabetes, insulin resistance diabetes, non-insulin-dependent diabetes, adult-onset diabetes, prediabetes; and Gestational Diabetes.

In summary, the invention described in this specification generally relates to dosage forms of L-4-chlorokynurenine and methods for treating various pathological conditions and disorders, such as neurological conditions characterized by overactive glutamatergic transmission mediated by N-methyl-D-aspartate receptors, as described above. These conditions include Major Depressive Disorder, OCD and tinnitus, as well as others disclosed above and other conditions as will be known to persons skilled in the art. Additionally, the invention also relates to methods of using L-4-chlorokynurenine in combination with various other compositions useful in treating various types of disorders, such as depression, including agents that augment the anti-depressive activity of other compositions. In another embodiment, the invention relates to personalized diagnostic assessment of a patient's likelihood to benefit from the administration of L-4-chlorokynurenine as a treatment of depressions (including MDD) based on measured CNS levels of kynurenic acid (KYNA) and quinolinic acid (QUIN).

While certain exemplary embodiments have been described above in detail and shown in the accompanying drawing figures, it is to be understood that such embodiments are merely illustrative of and not restrictive of the broad invention. In particular, it should be recognized that the teachings of the invention apply to a wide variety of diseases. Persons of skill in the art will recognize that various modifications may be made to the illustrated and other embodiments of the invention described above, without departing from its broad inventive scope. Thus, it will be understood that the invention is not limited to the particular embodiments or arrangements disclosed, but is rather intended to cover any changes, adaptations or modifications which are within the scope and spirit of the invention as defined by the appended claims.

### References:

The following journal articles and all other publications, patents and documents mentioned in this specification or listed below are incorporated by reference in their entirety.
Aitken, R.C., (1969) Measurement of feelings using visual analogue scales. Proc R Soc Med 62(10):989-93.
Akinfiresoye, L. and Y. Tizabi, (2013) Antidepressant effects of AMPA and ketamine combination: role of hippocampal BDNF, synapsin, and mTOR. Psychopharmacology (Berl) 230(2):291-8.
Amiel, S.A., et al., (2008) Hypoglycaemia in Type 2 diabetes. Diabet Med 25(3):245-54.
Axelsson, A. and A. Ringdahl, (1989) Tinnitus--a study of its prevalence and characteristics. Br J Audiol 23(1):53-62.
Beck, A.T. and A. Beamesderfer, (1974) Assessment of depression: the depression inventory. Mod Prob1 Pharmacopsychiatry 7(0):151-69.
Bay-Richter, C., et al. (2015) "A role for inflammatory metabolites as modulators of the glutamate N-methyl-d-aspartate receptor in depression and suicidality." Brain Behav Immun 43:110-117.
Bremner, J.D., et al., (1998) Measurement of dissociative states with the Clinician-Administered Dissociative States Scale (CADSS). J Trauma Stress 11(1):125-36.
Burris, R.E. and M. Hebrok, (2007) Pancreatic innervation in mouse development and beta-cell regeneration. Neuroscience 150(3):592-602.
Carter AJ. Glycine antagonist: Regulation of the NMDA receptor channel complex by the strychnine-insensitive glycine site. Drugs Future 1992;17:595-613.
Calabrese et al., 2012 "Stress-Induced Changes of Hippocampal NMDA Receptors: Modulation by Duloxetine Treatment," PLoS ONE 2012, 7(5): e37916. doi:10.1371/journal.pone.0037916
Catarzi et al., Competitive Gly/NMDA receptor antagonists, Curr. Top. Med. Chem. 2006;6(8):809-21.
Cronlein, T., et al., (2007) Tinnitus and insomnia. Prog Brain Res 166:227-33.
Erhardt, S., et al. (2013) "Connecting inflammation with glutamate agonism in suicidality." Neuropsychopharmacology 38:743-752.
Guitton, M.J., et al., (2003) Salicylate induces tinnitus through activation of cochlear NMDA receptors. J Neurosci 23(9):3944-52.
Guitton, M.J. and Y. Dudai, (2007) Blockade of cochlear NMDA receptors prevents long-term tinnitus during a brief consolidation window after acoustic trauma. Neural Plast 2007:80904.
Group, T.S., (2013) Effects of metformin, metformin plus rosiglitazone, and metformin plus lifestyle on insulin sensitivity and beta-cell function in TODAY. Diabetes Care 36(6):1749-57.
Hainer, V., (2014) Overview of new antiobesity drugs. Expert Opin Pharmacother 15(14):1975-8.
Hallam, R.S., S.C. Jakes, and R. Hinchcliffe, (1988) Cognitive variables in tinnitus annoyance. Br J Clin Psychol 27 ( Pt 3):213-22.
Hamilton, M., (1960) A rating scale for depression. J Neurol Neurosurg Psychiatry 23:56-62.
Hamilton, M., (1959) The assessment of anxiety states by rating. Br J Med Psychol 32(1):50-5.
Hokari M, Wu H-Q, Schwarcz R, Smith QR. Facilitated brain uptake of 4-chlorokynurenine and conversion to 7-chlorokynurenic acid. Neuroreport 1996;8(1): 15-18.
Hori, H. and H. Kunugi, (2012) The efficacy of pramipexole, a dopamine receptor agonist, as an adjunctive treatment in treatment-resistant depression: an open-label trial. ScientificWorldJournal 2012:372474.
Hunter, A.M., et al., (2011) The antidepressant treatment response index and treatment outcomes in a placebo-controlled trial of fluoxetine. J Clin Neurophysiol 28(5):478-82.
Ibrahim, L., et al., (2012) A Randomized, placebo-controlled, crossover pilot trial of the oral selective NR2B antagonist MK-0657 in patients with treatment-resistant major depressive disorder. J Clin Psychopharmacol 32(4):551-7.
IDF. Diabetes Atlas, Sixth edition 2013.
IDF. Diabetes Atlas, 6th edition. 2014; Available from: http://www.idf.org/diabetesatlas/update-2014.
Inagaki, N., et al., (1995) Expression and role of ionotropic glutamate receptors in pancreatic islet cells. FASEB J 9(8):686-91.
Ising, M., et al., (2007) Combined dexamethasone/corticotropin releasing hormone test predicts treatment response in major depression - a potential biomarker? Biol Psychiatry 62(1):47-54.
Ji, Y., et al., (2011) Glycine and a glycine dehydrogenase (GLDC) SNP as citalopram/escitalopram response biomarkers in depression: pharmacometabolomics-informed pharmacogenomics. Clin Pharmacol Ther 89(1):97-104.
Kamijima, K., et al., (2013) Aripiprazole augmentation to antidepressant therapy in Japanese patients with major depressive disorder: a randomized, double-blind, placebo-controlled study (ADMIRE study). J Affect Disord 151(3):899-905.
Kemp JA, Foster AC, Leeson PD, Priestley T, Tridgett R, Iversen LL, et al. 7-Chlorokynurenic acid is a selective antagonist at the glycine modulatory site of the N-methyl- D-aspartate receptor complex. Proc Natl Acads Sci U.S.A. 1988;85(17):6547-6550.
Kraemer, H.C., et al., (2002) Mediators and moderators of treatment effects in randomized clinical trials. Arch Gen Psychiatry 59(10):877-83.
Kuwabara, T., et al., (2011) Insulin biosynthesis in neuronal progenitors derived from adult hippocampus and the olfactory bulb. EMBO Mol Med 3(12):742-54.
Langguth, B., et al., (2007) Tinnitus severity, depression, and the big five personality traits. Prog Brain Res 166:221-5.
Lee S-C, Schwarcz R. Excitotoxic injury stimulates pro-drug-induced 7-chlorokynurenate formation in the rat striatum in vivo. Neuroscience Lett 2001;304(3):185-188.
Leeson PD, Iversen LL. The glycine site on the NMDA receptor: Structure-activity relationships and therapeutic potential. J Med Chem 1994;37(24):4053-4067.
Linderholm, et al., Activation of rat ventral tegmental area dopamine neurons by endogenous kynurenic acid: a pharmacological analysis, Neuropharmacology 2007;53(8):918-924.
Marquard, J., et al. (2015) "Characterization of pancreatic NMDA receptors as possible drug targets for diabetes treatment." Nat Med 21:363-372.
Macfadden, W., et al., (2011) Adjunctive long-acting risperidone in patients with bipolar disorder who relapse frequently and have active mood symptoms. BMC Psychiatry 11:171.
Montgomery, S.A. and M. Asberg, (1979) A new depression scale designed to be sensitive to change. Br J Psychiatry 134:382-9.
Musselman, D. et al. (1988). "The relationship of depression to cardiovascular disease: epidemiology, biology, and treatment." Arch. Gen. Psychiatry 55(7): 580-592 (1988).
Myint, A. M., et al. (2007) "Kynurenine pathway in major depression: evidence of impaired neuroprotection." J Affect Disord 98:143-151.
Nondahl, D.M., et al., (2007) The impact of tinnitus on quality of life in older adults. J Am Acad Audiol 18(3):257-66.
Oestreicher, E., et al., (1998) Memantine suppresses the glutamatergic neurotransmission of mammalian inner hair cells. ORL J Otorhinolaryngol Relat Spec 60(1):18-21.
Overall, J.E. and D.R. Gorham, (1962) The Brief Psychiatric Rating Scale. Psychological Reports 10(3):799-812.
Parsons CG, Danysz W, Quack G, Hartmann S, Lorenz B, Wollenburg C, et al. Novel systemically active antagonists of the glycine site of the N-methyl-D-aspartate receptor: electrophysiological, biochemical and behavioral characterization. J Pharmacol Exp Ther 1997;283(3): 1264-1275.
Peng, B.G., S. Chen, and X. Lin, (2003) Aspirin selectively augmented N-methyl-D-aspartate types of glutamate responses in cultured spiral ganglion neurons of mice. Neurosci Lett 343(1):21-4.
Posner K., et al., (2011) The Columbia-Suicide Severity Rating Scale: initial validity and internal consistency findings from three multisite studies with adolescents and adults. Am J Psychiatry 168(12):1266-77.
Quante, A., et al., (2013) Quetiapine as combination treatment with citalopram in unipolar depression with prominent somatic symptoms: a randomised, double-blind, placebo-controlled pilot study. Psychiatr Danub 25(3):214-20.
Rao TS, Gray NM, Dappen MS, Cler JA, Mick SJ, Emmett MR, et al. Indole-2-carboxylates, novel antagonists of the N-methyl-D-aspartate (NMDA)-associated glycine recognition sites: in vivo characterization. Neuropharmacol 1993;32(2): 139-147.
Rodriguez-Diaz, R., et al., (2012) Real-time detection of acetylcholine release from the human endocrine pancreas. Nat Protoc 7(6):1015-23.
Rosenstock, J., et al., (2014) Beneficial effects of once-daily lixisenatide on overall and postprandial glycemic levels without significant excess of hypoglycemia in type 2 diabetes inadequately controlled on a sulfonylurea with or without metformin (GetGoal-S). J Diabetes Complications 28(3):386-92.
Rundfeldt C , Wlaz P , Loscher W . Anticonvulsant activity of antagonists and partial agonists for the NMDA receptor-associated glycine site in the kindling model of epilepsy. Brain Res 1994;653(1-2): 125-130.
Salituro et al., (1994) Enzyme-Activated Antagonists of the Strychnine-Insensitive Glycine/NMDA Receptor, J. Med. Chem. 1994:37-334,336.
Schwartz, M.W., et al., (2013) Cooperation between brain and islet in glucose homeostasis and diabetes. Nature 503(7474):59-66.
Shi, Y. and F.B. Hu, (2014) The global implications of diabetes and cancer. Lancet 383(9933):1947-8.
Sepanjnia, K., et al., (2012) Pioglitazone adjunctive therapy for moderate-to-severe major depressive disorder: randomized double-blind placebo-controlled trial. Neuropsychopharmacology 37(9):2093-100.
Siegle, G.J., et al., (2012) Toward clinically useful neuroimaging in depression treatment: prognostic utility of subgenual cingulate activity for determining depression outcome in cognitive therapy across studies, scanners, and patient characteristics. Arch Gen Psychiatry 69(9):913-24.
Skrivanek, Z., et al., (2014) Dose-finding results in an adaptive, seamless, randomized trial of once-weekly dulaglutide combined with metformin in type 2 diabetes patients (AWARD-5). Diabetes Obes Metab 16(8):748-56.
Soltani, N., et al., (2011) GABA exerts protective and regenerative effects on islet beta cells and reverses diabetes. Proc Natl Acad Sci U S A 108(28):11692-7.
Tambasco et al. (2012) N. et al "Clinical Aspects and Management of Levodopa-Induces Dyskinesia," Parkinson's Disease, Article ID 745947, doi:10.1 155/2012/745947.
Tasyurek, H.M., et al., (2014) Incretins: their physiology and application in the treatment of diabetes mellitus. Diabetes Metab Res Rev 30(5):354-71.
Tyler, R.S. and D. Conrad-Armes, (1983) The determination of tinnitus loudness considering the effects of recruitment. J Speech Hear Res 26(1):59-72.
Van Hook, M.P., B. Berkman, and R. Dunkle, (1996) Assessment tools for general health care settings: PRIME-MD, OARS, and SF-36. Primary Care Evaluation of Mental Health Disorders. Older Americans Resources and Services Questionnaire; Short Form-36. Health Soc Work 21(3):230-4.
Vetterli, L., et al., (2012) Delineation of glutamate pathways and secretory responses in pancreatic islets with beta-cell-specific abrogation of the glutamate dehydrogenase. Mol Biol Cell 23(19):3851-62.
Vio, M.M. and R.H. Holme, (2005) Hearing loss and tinnitus: 250 million people and a US$10 billion potential market. Drug Discov Today 10(19): 1263-5.
Vos, T., et al., (2012) Years lived with disability (YLDs) for 1160 sequelae of 289 diseases and injuries 1990-2010: a systematic analysis for the Global Burden of Disease Study 2010. Lancet 380(9859):2163-96.
WHO. The top 10 causes of death Fact sheet. 2013; Fact sheet N°310: [Available from: http://www.who.int/mediacentre/factsheets/fs310/en/.
WHO. Diabetes Fact Sheet, N 312. 2015; Available from: http://www.who.int/mediacentre/factsheets/fs312/en/.
□ Wolkowitz, O.M., et al., (2012) Resting leukocyte telomerase activity is elevated in major depression and predicts treatment response. Mol Psychiatry 17(2):164-72.
Wu HQ, Lee SC, Scharfman HE, Schwarcz R. L-4-chlorokynurenine attenuates kainate-induced seizures and lesions in the rat. Exp Neurol 2002;177(1):222-232.
Wu H-Q, Lee S-C, Schwarcz R. Systemic administration of 4-chlorokynurenine prevents quinolinate neurotoxicity in the rat hippocampus. Eur J Pharm 2000;390:267-274.
Young, R.C., et al., (1978) A rating scale for mania: reliability, validity and sensitivity. Br J Psychiatry 133:429-35.
Yang, W., et al., (2013) Economic costs of diabetes in the U.S. in 2012. Diabetes Care 36(4): 1033-46.
Zarate, C.A., Jr., et al., (2013) A randomized trial of a low-trapping nonselective N-methyl-D-aspartate channel blocker in major depression. Biol Psychiatry 74(4):257-64.
Zarate, C.A.J., et al., (2005) An open-label trial of the glutamate-modulating agent riluzole in combination with lithium for the treatment of bipolar depression. Biol Psychiatry 57:430-432.
Zarate, C.A., Jr., et al., (2006) A randomized trial of an N-methyl-D-aspartate antagonist in treatment-resistant major depression. Arch Gen Psychiatry 63(8):856-64.
U.S. Patent No. 5,547,991 to Palfreyman et al.
Published international patent application WO/2014/152752.
Published international patent application WO/2014/152835.

### FURTHER EMBODIMENTS

1. A method of treating tinnitus by administering an effective dose of L-4-chlorokynurenine.
2. A method of treating obsessive-compulsive disorder by administering an effective dose of L-4-chlorokynurenine.
3. The method of any of embodiments 1 to 2, wherein the pharmaceutical composition is administered in a daily dose or intermittent dose of from one to about 30 days.
4. The method of any of embodiments 1 to 2, wherein the effective amount of L-4-chlorokynurenine is administered in a range of about 50 mg to about 1,800 mg per day.
5. The method of embodiment 5, wherein the pharmaceutical composition consists essentially of L-4-chlorokynurenine in an amount from the group comprising about 360, 1,080 or 1,440 mg, together with pharmaceutically acceptable ingredients such as carriers and excipients.
6. A pharmaceutical composition comprising L-4-chlorokynurenine packaged with a label indicating the use of this composition for the treatment of a condition selected from the group comprising tinnitus and obsessive-compulsive disorder.

## Claims

1. L-4-chlorokynurenine for use in a method for treating tinnitus.

2. L-4-chlorokynurenine for the use of claim 1, wherein the effective dose of L-4-chlorokynurenine is administered ranges from about 50 mg to about 1,800 mg per day or is an amount of 360, 1,080 or 1,440 mg per day; and wherein the L-4-chlorokynurenine is optionally administered together with a pharmaceutically acceptable carrier or excipient.

3. A pharmaceutical composition comprising a therapeutically acceptable amount of L-4-chlorokynurenine packaged with a label indicating the use of the composition for the treatment of tinnitus.

4. L-4-chlorokynurenine for use in a method for treating obsessive-compulsive disorder.

5. L-4-chlorokynurenine for the use of claim 4, wherein the effective dose of L-4-chlorokynurenine is administered ranges from about 50 mg to about 1,800 mg per day or is an amount of 360, 1,080 or 1,440 mg per day; and wherein the L-4-chlorokynurenine is optionally administered together with a pharmaceutically acceptable carrier or excipient.

6. A pharmaceutical composition comprising a therapeutically acceptable amount of L-4-chlorokynurenine packaged with a label indicating the use of the composition for the treatment of obsessive-compulsive disorder.

7. L-4-chlorokynurenine for use in a method for treating major depressive disorder.

8. L-4-chlorokynurenine for use of claim 7, wherein the effective dose of L-4-chlorokynurenine is administered ranges from about 50 mg to about 1,800 mg per day or is an amount of 360, 1,080 or 1,440 mg per day; and wherein the L-4-chlorokynurenine is optionally administered together with a pharmaceutically acceptable carrier or excipient.

9. A pharmaceutical composition comprising a therapeutically acceptable amount of L-4-chlorokynurenine packaged with a label indicating the use of the composition for the treatment of major depressive disorder.

10. L-4-chlorokynurenine for use in a method for treating diabetes.

11. L-4-chlorokynurenine for the use of claim 10, wherein the effective dose of L-4-chlorokynurenine is administered ranges from about 50 mg to about 1,800 mg per day or is an amount of 360, 1,080 or 1,440 mg per day; and wherein the L-4-chlorokynurenine is optionally administered together with a pharmaceutically acceptable carrier or excipient.

12. A pharmaceutical composition comprising a therapeutically acceptable amount of L-4-chlorokynurenine packaged with a label indicating the use of the composition for the treatment of diabetes.

13. L-4-chlorokynurenine for use in a method for treating depression by co-administering an effective dose of L-4-chlorokynurenine with a second antidepressant therapy.

14. L-4-chlorokynurenine for the use of claim 13, wherein the effective dose of L-4-chlorokynurenine is administered ranges from about 50 mg to about 1,800 mg per day or is an amount of 360, 1,080 or 1,440 mg per day; and wherein the L-4-chlorokynurenine is optionally administered together with a pharmaceutically acceptable carrier or excipient.

15. A pharmaceutical composition comprising a therapeutically acceptable amount of L-4-chlorokynurenine packaged with a label indicating the use of the composition for the treatment of depression of claim 13.
